# EUROPÄISCHE PATENTSCHRIFT

(11) **EP 2 526 973 B1**
(45) Veröffentlichungstag und Bekanntmachung des Hinweises auf die Patenterteilung: **21.06.2017**
(21) Anmeldenummer: 12181686.2
(22) Anmeldetag: 12.09.2007
(51) Int. Cl.: A61K 49/04

(54) **Röntgenkontrastmittel für die postmortale, experimentelle und diagnostische Angiographie**
X ray contrasting agent for post-mortem, experimental and diagnostic angiography
Produit de contraste radiographique pour l'angiographie post-mortem, expérimentale et diagnostique

(30) Priorität: 18.09.2006 CH 14852006; 08.02.2007 CH 2122007
(43) Veröffentlichungstag der Anmeldung: 28.11.2012
(62) Teilanmeldung aus: 07800637.6
(73) Patentinhaber: Forim-X AG, 5630 Muri (CH)
(72) Erfinder: Grabherr, Silke, 1026 Denges (CH); Gygax, Erich, 3097 Liebefeld (CH)
(74) Vertreter: Bremi, Tobias Hans

(56) Entgegenhaltungen:
- WO-A-92/18169
- WO-A-97/24064
- DE-B1- 2 602 907
- GB-A- 721 264
- GB-A- 2 014 043
- US-A- 2 675 343
- SAVAGE LABORATORIES: "Ethiodol (ethiodized oil) Injection", DAILY MED - INTERNET ARTICLE , XP002431180, Gefunden im Internet: URL:http://dailymed.nlm.nih.gov/dailymed/d rugInfo.cfm?id=1812 [gefunden am 2007-04-21]
- GRABHERR S. ET AL.: "Postmortem Angiography: Review of former and current Methods", FORENSIC RADIOLOGY REVIEW, Bd. 188, März 2007 (2007-03), Seiten 832-838, XP008078071,
- GATES CORPORATION: "Viscosity Data", INTERNET ARTICLE , XP002431181, Gefunden im Internet: URL:http://www.gates.com/brochure.cfm?broc hure=2625&location_id=3046 [gefunden am 2007-04-21]
- J. BERMEYER: "Postmortale Koronangiographie und Perfusion normaler und pathologisch veränderter Herzen, Messung der Durchflusskapazität interkoronarer Anastomosen", BEITR. PATH. ANAT., Bd. 137, 1968, Seiten 373-390, XP008078238,

## Beschreibung

### TECHNISCHES GEBIET

Die vorliegende Erfindung betrifft ein Kontrastmittel für die postmortale Angiographie, insbesondere zur Untersuchung von tierischen oder menschlichen Leichen oder Bestandteilen davon, z.B. von Gliedmassen oder von Organen. Das Kontrastmittel enthält dabei eine im wesentlichen ölbasierte, apolare Kontrastkomponente, wie sie für Röntgenuntersuchungen verwendet wird, wobei die Kontrastkomponente eine Kontrastkomponentenviskosität im Bereich von 30-100 mPas aufweist. Weiterhin betrifft die Erfindung angiographische Verfahren, in welchen ein derartiges Kontrastmittel wenigstens während Zeitabschnitten eingesetzt wird.

### STAND DER TECHNIK

Angiographie ist die Darstellung von Blutgefässen mittels Röntgenstrahlen. Hierzu wird ein Kontrastmittel, d. h. ein Stoff, der für Röntgenstrahlen kaum durchlässig ist, in das Blutgefäss injiziert. Auf dem Röntgenbild zeichnet sich dann der mit Kontrastmittel gefüllte Gefässinnenraum ab. Das resultierende Bild nennt man Angiogramm. In der Medizinersprache wird das lange Wort Angiografie oft mit dem Wort Angio abgekürzt. Zur Angiographie kann unter anderem auch Computertomographie eingesetzt werden.

Die Computertomographie, Abkürzung CT, ist die rechnergestützte Auswertung einer Vielzahl aus verschiedenen Richtungen aufgenommener Röntgenaufnahmen eines Objektes, um ein dreidimensionales Bild zu erzeugen (Voxeldaten). Es handelt sich dabei um ein bildgebendes Verfahren.

Die derzeit in Verwendung stehenden Techniken zur bildgebenden Gefässuntersuchung lassen sich in Gruppen von Methoden einteilen: Einerseits stehen Ausgusstechniken zur Verfügung, andererseits gibt es Kontrastmittel, die in der Lage sind Kapillaren zu penetrieren. Eine allgemeine Übersicht über sämtliche Techniken der postmortalen Angiographie kann in "Postmortem Angiography - A Review of Former and Current Methods" (S. Grabherr et al, AJR 2006 in press) gefunden werden.

Das nach dem Prioritätstag aber vor dem Anmeldetag der vorliegenden Anmeldung veröffentlichte Dokument von S. Grabherr. et al, FORENSIC RADIOLOGY REVIEW, Bd. 188, März 2007, Seiten 832-838, offenbart ein Kontrastmittel für die postmortale Angiographie, enthaltend eine 0.1 % Sudanrot-Diesel-Lösung und Lipiodol.

### Ausgusstechniken:

Bei den Ausgusstechniken werden beispielsweise Methylmethacrylate (zum Beispiel Mercox®), oder Microfil® eingesetzt. Diese Substanzen werden nach vorheriger Spülung der Gefässe frisch angemischt injiziert. Nach ihrer Aushärtung wird das Gewebe wegmazeriert, was 2 - 4 Wochen dauert. Übrig bleibt dann der Ausguss der Gefässe, der von blossem Auge, mit Hilfe eines Mikroskops oder Elektronenmikroskops oder auch mittels Röntgentechniken untersucht werden kann (siehe Djonov et al, Anal Embryo/ 2000;202:347-357). Nachteile dieser Methode sind die aufwendige Herstellung dieser Präparate, die lange Zeitdauer, die das Mazerieren benötigt und nicht zuletzt die hohe Gefahr von Beschädigungen der Präparate durch das Mazerieren und das anschliessende Hantieren, bei dem sehr leicht kleine Stückchen des Präparates abbrechen können.

### Flüssige Kontrastmittel:

Die meisten flüssigen Konstrastmittel eignen sich nicht für Mikroangiographien. Auf Wasser basierende Mischungen haben die Eigenschaft, sehr schnell die Gefässe durch Penetration durch die Gefässwand zu verlassen (sogenannte Extravasation), was zu verwaschenen Konturen der dargestellten Gefässe führt. Auch ist ihr Kontrast nicht hoch genug, um kleine Gefässe wie Kapillaren klar darzustellen.

Ölige Kontrastmittel haben die Eigenschaft, innerhalb der Gefässe zu bleiben, ohne die Gefässwand zu penetrieren (normalerweise keine Extravasation). Dies führt zu einer deutlich konturierten Darstellung der Gefässe. Jedoch besteht keine Kapillargängigkeit aufgrund auftretender Verstopfungen der sogenannten Haargefässe.

Korpuskuläre Kontrastmittel können bei Verwendung kleiner korpuskulärer Partikel zur Microangiographie verwendet werden. Das bekannteste Kontrastmittel ist Micropaque ®. Bei seiner Verwendung in Kombination mit Micro-CT-Geräten wurde jedoch das Problem einer Ausfällung der gelösten Partikel beschrieben, was zu Artefakten führt (M Marxen et al, Med Phys 2004,31:305-313). Marxen beschreibt in seinem Paper klar die Notwendigkeit und das Fehlen eines geeigneten Kontrastmittels für Microangiographie mittels Micro-CT.

### DARSTELLUNG DER ERFINDUNG

Der Erfindung liegt demnach die Aufgabe zugrunde, ein verbessertes Kontrastmittel für bildgebende Verfahren der eingangs genannten Art zur Verfügung zu stellen. Insbesondere geht es dabei also um die Verbesserung eines Kontrastmittels für die Angiographie, beispielsweise zur Untersuchung von tierischen oder menschlichen Leichen oder Bestandteilen davon, z.B. von Gliedmassen (welche auch noch am Körper sein können) enthaltend eine im wesentlichen apolare (mit anderen Worten so gut wie nicht oder gar nicht mit Wasser mischbare), das heisst in der Regel ölbasierte, Kontrastkomponente für Röntgenuntersuchungen. Die Kontrastkomponente verfügt dabei bei diesem Typ über eine relativ hohe Viskosität, typischerweise im Bereich von 30-100 mPas.

Problematisch an solchen apolaren öligen Kontrastkomponenten ist die Tatsache, dass sie eine hohe Viskosität aufweisen. Diese hohe Viskosität führt dazu, dass die Kontrastkomponente nicht beliebig in Kapillaren eindringen kann und entsprechend auch nicht das Blutgefässsystem vollständig abzubilden in der Lage ist. Zudem kann es sogar zu einer richtiggehenden Verstopfung von Kapillaren und Abschnitten führen. Der Vorteil der im wesentlichen nicht vorhandenen Extravasation wird dadurch bei vielen Anwendungen wieder rückgängig gemacht.

Überraschenderweise wurde festgestellt, dass es bei derartigen Kontrastkomponenten aber möglich ist, diese mit grossem Vorteil in einer Mischung zu verwenden. Die Mischung ist dabei dadurch gekennzeichnet, dass die Kontrastkomponente in einer Mischung mit wenigstens einer weiteren apolaren Komponente, deren Viskosität geringer ist oder höchstens gleich gross wie die Kontrastkomponentenviskosität, vorliegt. Die Zumischung dieser weiteren apolaren Komponente wird einerseits dazu verwendet werden, die Viskosität auf den gewünschten Wert, beispielsweise auf den Wert von Blut, einzustellen. Andererseits wird sie dazu verwendet, als Träger für die Kontrastkomponente zu wirken, ohne deren Eigenschaften hinsichtlich der Extravasation zu verändern. Unerwartet war dies insbesondere hinsichtlich der Tatsache, dass die positiven Eigenschaften bezüglich Extravasation beibehalten werden können und trotzdem die Viskosität der gesamten Mischung auf einen optimalen Wert eingestellt werden kann, und trotz Verdünnung der Kontrastkomponente ein hervorragender Kontrast möglich ist.

Bevorzugtermassen ist das Kontrastmittel im wesentlichen vollständig frei von Wasser, das heisst es liegt nicht als Emulsion vor. Es handelt sich mit anderen Worten bevorzugtermassen bei der erfindungsgemässen Mischung um eine Mischung, welche nur aus apolaren Komponenten besteht, wobei diese Komponenten bevorzugtermassen untereinander im wesentlichen beliebig mischbar sind.

Eine erste bevorzugte Ausführungsform ist dadurch gekennzeichnet, dass die weitere apolare Komponente ein im wesentlichen gesättigter, verzweigter, unverzweigter, oder zyklischer Kohlenwasserstoff ist, oder ein Gemisch derartiger Kohlenwasserstoffe. Bevorzugtermassen ist die weitere apolare Komponente ein Alkan (bevorzugtermassen n-Alkan, iso-Alkan) oder ein Gemisch von Alkanen, mit einer Anzahl von Kohlenstoffatomen im Bereich von 5 - 45, insbesondere bevorzugt im Bereich von 12 - 30.

Beispielsweise ist es möglich, eine apolare Komponente mit einer vergleichsweise niedrigen Viskosität beizumischen, so beispielsweise ein Alkan mit 12-18, bevorzugt mit 14-16 Kohlenstoffatomen, oder um ein Gemisch von derartigen Alkanen, wobei es sich dabei insbesondere bevorzugt um ein n-Alkan oder ein Gemisch von solchen n-Alkanen handelt. Bevorzugt werden so insbesondere Tetradekan oder Hexadekan, es können aber auch Systeme wie Cyclohexan oder Gemische wie beispielsweise Petroleum oder sogar Petrolether eingesetzt werden. Bevorzugte Systeme besitzen einen Schmelzpunkt oberhalb von 0°C und/oder einen Siedepunkt von wenigstens 200°C.

Eine weitere bevorzugte Ausführungsform ist dadurch gekennzeichnet, dass es sich bei der weiteren apolaren Komponente um eine Komponente mit einer eher höheren Viskosität (aber immer noch höchstens gleich gross wie jene der Kontrastkomponente) handelt, so beispielsweise um ein Alkan, insbesondere um ein n-Alkan oder auch ein iso-Alkan, mit 20 - 30 Kohlenstoffatomen. Erfindungsgemäss handelt es sich um Paraffinöl, insbesondere bevorzugt um paraffinum perliquidum. Die apolare Komponente mit einer eher höheren Viskosität kann aber auch ein pflanzliches Öl sein, so beispielsweise ein methyliertes Rapsöl oder ähnliche pflanzliche Öle.

Eine zusätzliche bevorzugte Ausführungsform zeichnet sich dadurch aus, dass die Kontrastkomponente eine Viskosität im Bereich von 30 - 80 mPas aufweist. So handelt es sich für die Angiographie beispielsweise bei der Kontrastkomponente bevorzugtermassen um ein iodbasiertes oder schwefelbasiertes Röntgenkontrastmittel, insbesondere bevorzugt um eine iodierte oder bromierte Komponente oder insbesondere Öl, beispielsweise um ein Propyliodon, einen Fettsäureethylester von iodiertem Mohnöl (z.B. Lipiodol), oder Iodipin, wobei es sich insbesondere bevorzugt um Lipiodol ultrafluid handelt.

Eine ganz besonders bevorzugte Ausführungsform der Mischung kennzeichnet sich dadurch, dass die weitere apolare Komponente eine Viskosität aufweist, welche niedriger ist als die Kontrastkomponentenviskosität. So verfügt bevorzugtermassen die weitere apolare Komponente über eine Viskosität im Bereich von 0.2 - 80 mPas, bevorzugt im Bereich von 2 - 60 mPas.

Wie bereits erläutert, ist es möglich, eine apolare Komponente mit einer vergleichsweise niedrigen Viskosität beizumischen, so bevorzugt mit einer Viskosität im Bereich von 1 - 10 mPas, bevorzugt im Bereich von 2 - 4 mPas. Alternativ oder insbesondere bevorzugt zusätzlich (als z.B. ternäre oder sogar multinäre Mischung) ist es möglich, eine apolare Komponente mit einer eher höheren Viskosität (immer noch niedriger als die Kontrastkomponentenviskosität) beizumischen, so beispielsweise mit einer Viskosität im Bereich von 25-80 mPas, wobei es sich, wie bereits erläutert, dabei insbesondere bevorzugt um paraffinum perliquidum handelt.

Besonders bevorzugt wird also beispielsweise eine binäre Mischung aus Lipiodol ultrafluide, und Tetradecan oder Hexadecan, oder erfindungsgemäss eine binäre Mischung aus Lipiodol ultrafluide und paraffinum perliquidum, oder eine ternäre Mischung aus Lipiodol ultrafluide, Tetradecan oder Hexadecan, und paraffinum perliquidum.

Bevorzugtermassen ist das Kontrastmittel dabei dadurch gekennzeichnet, dass das Volumen-Verhältnis von Kontrastkomponente zu weiterer apolarer Komponente im Bereich von 1:1 bis 1:10, bevorzugt im Bereich von 1:2 - 1:8, insbesondere bevorzugt im Bereich von 1:4 - 1:6 oder im Bereich von 1:2-1:5 liegt.

Dies ist bei der Angio-Injektionsmethode und der Angio-Perfusuinsmethode bevorzugtermassen der Bereich für das Volumen-Verhältnis einer Kontrastkomponente zu einer weiteren apolaren Komponente, welche eine eher höhere Viskosität (beispielsweise 25-80 mPas, vergleiche Spezifikationen oben, entspricht z.B. einem Alkan, insbesondere einem n-Alkan oder auch einem iso-Alkan, mit 20 - 30 Kohlenstoffatomen wie z.B. Paraffinöl, insbesondere bevorzugt paraffinum perliquidum) aufweist. Das Volumen-Verhältnis von Kontrastkomponente (oder von Kontrastkomponente bereits in einer Mischung mit einer weiteren apolaren Komponente, welche eine eher höhere Viskosität aufweist) zu einer weiteren apolaren Komponente mit einer vergleichsweise niedrigen Viskosität (beispielsweise im Bereich von 1-10 mPas, vergleiche Spezifikationen oben, entspricht zum Beispiel einem Alkan mit 12-18, bevorzugt mit 14-16 Kohlenstoffatomen, oder einem Gemisch von derartigen Alkanen, insbesondere bevorzugt einem n-Alkan oder einem Gemisch von solchen n-Alkanen, so z.B. insbesondere Tetradekan oder Hexadekan) stellt man bei der Angio-Injektionsmethode und der Angio-Perfusuinsmethode am besten unter Berücksichtigung der gewünschten Viskosität ein, was beispielsweise Verhältnisse (Kontrastkomponente : apolare Komponente mit einer vergleichsweise niedrigen Viskosität) im Bereich von 20:1 - 100:1 oder bevorzugtermassen 30 : 1 - 70:1 ergibt.

Für die so genannte Angio-Injektionsmethode wird bevorzugtermassen eine ternäre Mischung eingesetzt, wobei die Kontrastkomponente (z.B. Lipiodol) und eine apolare Komponente mit einer eher höheren Viskosität (z.B. paraffinum perliquidum, gewissermassen als Träger) in einem Verhältnis im Bereich von 1:5 eingesetzt wird, und anschliessend die Komponente mit einer vergleichsweise niedrigen Viskosität (z.B. Hexadecan und/oder Tetradecan) z.B. in einem Verhältnis 50:1 eingesetzt wird, um die Viskosität der gesamten Mischung wiederum in Abhängigkeit des Röntgenkontrasts und des gewünschten Fliessverhaltens einzustellen.

Insbesondere für Microangio werden dabei Mischungen aus Kontrastkomponente und apolarer Komponente mit einer vergleichsweise niedrigen Viskosität (z. B. Tetradecan oder Hexadecan) eingesetzt in einem Verhältnis von 1: 4 oder 1: 6, wobei die Menge apolarer Komponente über den gewünschten Röntgenkontrast und die Eindringtiefe in kleine Kapillaren eingestellt wird.

Des Weiteren betrifft die vorliegende Erfindung ein Verfahren zur dynamischen angiographischen Untersuchung von tierischen oder menschlichen Leichen oder Organen, Bestandteilen, z.B. von Gliedmassen, davon. Das Verfahren ist bevorzugtermassen dadurch gekennzeichnet, dass ein Kontrastmittel, z.B. wie es oben beschrieben wurde, in das Blutgefässsystem eingebracht wird und anschliessend oder synchron eine Aufnahme unter Zuhilfenahme von Röntgenstrahlen, insbesondere eine CT-Aufnahme, vorgenommen wird.

Zudem betrifft die vorliegende Erfindung ein gewissermassen dynamisches Verfahren zur angiographischen Untersuchung von tierischen oder menschlichen Leichen oder Organen, Bestandteilen, z.B. von Gliedmassen davon. Dieses Verfahren ist dadurch gekennzeichnet, dass zunächst eine weitere apolare Komponente, wie sie oben geschildert wurde in das Blutgefässsystem im wesentlichen kontinuierlich eingebracht und darin zirkuliert wird, und während eines Zeitabschnitts dieser apolaren Komponente eine Kontrastkomponente, wie sie oben charakterisiert wurde, beigemischt wird, wobei anschliessend, synchron oder abschnittsweise Aufnahmen unter Zuhilfenahme von Röntgenstrahlen, insbesondere eine CT-Aufnahme, vorgenommen werden (so genannte dynamische Angiographie, zum Beispiel zunächst arterielle Bildgebung, dann parenchymale Bildgebung und dann venöse Bildgebung, wobei als Träger paraffinum perliquidum, ggf. in Mischung mit einem Decan (unter dem Begriff Decan sei in der Folge Tetradecan und/oder Hexadecan zu verstehen), eingesetzt wird und ein Stoss von Kontrastkomponente eingebracht wird). Generell ist es übrigens auch möglich, die weitere Komponente(n) einzufärben, so z.B. ist eine Rotfärbung des paraffinum perliquidum mit Sudanrot möglich.

Das Kontrastmittel zur Verwendung in diesem Verfahren kann also gemäss einer ersten bevorzugten Ausführungsform ein apolares Gemisch sein, wie es im Detail oben erläutert wurde. Es kann aber auch ein im wesentlichen polares Gemisch sein, welches bevorzugtermassen auf wässriger Basis ist, und bei welchem dann auch eine in Wasser lösliche Kontrastkomponente oder einer in Wasser emulgierbare Kontrastkomponente verwendet wird. Zur Einstellung der Viskosität einer solchen Mischung als Kontrastmittel können polare Komponenten mit hoher Viskosität beigemischt werden. Solche Komponenten können beispielsweise Polyole wie z.B. langkettige Zucker oder langkettige Glykole, sein wie z.B. Polyethylenglykol (z.B. PEG 200 von Schärer und Schläpfer AG, Rothrist, CH). Als Kontrastkomponente kann dann z.B. Imagopaque 300 (Iopentol, Amersham Health) verwendet werden. Auch hier kann die gewünschte Viskosität des Kontrastmittels entsprechend dann eingestellt werden durch die zusätzliche polare Komponente respektive deren Anteil im Wasser. Typischerweise wird dabei eine Viskosität des Kontrastmittels im Bereich von 0.5 - 60 mPas, insbesondere bevorzugt im Bereich von 0.1-50 mPas, besonders bevorzugt im Bereich von 20- 45 oder 30 - 40 mPas, eingestellt für die erfindungsgemässen Anwendungen. In diesem Fall besteht die Mischung also bevorzugtermassen aus einer im wesentlichen polaren Kontrastkomponente (oder in Wasser emulgierbaren Kontrastkomponente) und einer weiteren polaren Komponente, wobei diese weitere polare Komponente entweder allein vorliegen kann, wenn dadurch bereits die gewünschte Viskosität erzielt werden kann, oder aber als Mischung. Liegt die weitere polare Komponente als Mischung vor, so wird bevorzugt ein System verwendet, bei welchem eine erste weitere polare Komponente mit einer niedrigen Viskosität, bevorzugtermassen Wasser, in Kombination mit einer zweiten weiteren polaren Komponente mit einer hohen Viskosität, bevorzugtermassen ein Polyol, eingesetzt wird. Dabei kann das Verhältnis zwischen der ersten weiteren polaren Komponente und der zweiten weiteren polaren Komponente dazu verwendet werden, die Viskosität des Kontrastmittels einzustellen. Diese Einstellung kann bevorzugtermassen dynamisch geschehen, dass heisst es ist möglich, genau wie bei Verwendung des oben im Detail beschriebenen apolaren Systems, im Laufe der Durchführung der Analyse die Viskosität durch Veränderung des Verhältnisses dieser beiden Komponenten unterschiedlich einzustellen und damit im Laufe der Analyse unterschiedliche Bereiche des Gefässsystems sichtbar zu machen.

Zudem betrifft die vorliegende Erfindung die Verwendung eines Kontrastmittels, wie es oben geschildert wurde, zur angiographischen Untersuchung von tierischen oder menschlichen Leichen oder Organen, Bestandteilen, z.B. von Gliedmassen davon. Gemäss einem weiteren Ausführungsbeispiel wird ein Verfahren vorgeschlagen, welches dadurch gekennzeichnet ist, dass ein Kontrastmittel, bevorzugtermassen in Form einer Mischung, wie die oben beschrieben wurde, in nicht pulsierender Weise in die Blutgefässe eingebracht wird. Sämtliche Messungen am lebenden Körper müssen in pulsierender Weise zirkuliert werden, was nun an einer Leiche nicht erforderlich ist und wesentliche zusätzliche analytische Möglichkeiten eröffnet. Bevorzugtermassen wird das Kontrastmittel also unter im wesentlichen konstantem oder zeitlich mit wesentlich weniger oder wesentlich mehr als der natürlich möglichen Herzfrequenz variierendem Druck in die Blutgefässe eingebracht. Bevorzugtermassen wird also das Zirkulationsmedium nicht volumenkontrolliert sondern druckkontrolliert eingebracht.

Ein weiterer grosser analytischer Zusatznutzen, welcher aus den natürlichen Analysen nicht verfügbar ist, ist die Möglichkeit, das Kontrastmittel im Blutgefässsystem wenigstens abschnittsweise im Prozess entgegen der natürlichen Flussrichtung des Blutes zu zirkulieren. Auch werden, gegebenenfalls in Kombination mit der genannten Drucksteuerung und der Variation weiterer Parameter, welche am lebenden Körper nicht derart variiert werden können, unglaubliche zusätzliche analytische Möglichkeiten eröffnet. Generell kann zudem die Differenz bezüglich Volumen, Druck, Temperatur und/oder Komponentenkonzentration aus in das Kreislaufsystem eingeführtem Kontrastmittel und aus aus dem Kreislaufsystem austretendem Kontrastmittel registriert und in der Analyse bevorzugtermassen in quantitativer Weise mit berücksichtigt werden. So kann beispielsweise der Blutverlust durch eine Öffnung im Kreislaufsystem quantitativ ermittelt werden.

Generell ist es zudem oder alternativ möglich, das Kontrastmittel bei der Einführung in das Kreislaufsystem in wenigstens einem der folgenden Parameter während und/oder vor der Messung, insbesondere bevorzugt in Abhängigkeit der aufgenommenen Bilddaten, zu variieren: Temperatur, Mischungsverhältnis, Druck. Vor der Einführung von Kontrastmittel kann das Kreislaufsystem insbesondere bevorzugt unter Verwendung einer apolaren Komponente oder einer (physiologischen) NaCl- oder Lactatlösung, gespült werden.

Des weiteren betrifft die vorliegende Erfindung eine Vorrichtung zur Durchführung eines Verfahrens, wie es oben beschrieben wurde. Diese Vorrichtung ist bevorzugtermassen dadurch gekennzeichnet, dass sowohl Mittel zur pumpengetriebenen Einbringung von Kontrastmittel in das Kreislaufsystem vorgesehen sind als auch Mittel zur Aufnahme und quantitativen Erfassung des nach Zirkulation aus dem Kreislaufsystem austretenden Kontrastmittels.

Alternativ oder zusätzlich kann die Vorrichtung dadurch gekennzeichnet sein, dass eine Einheit angeordnet ist, mit welcher vor der Einbringung des Kontrastmittels in das Kreislaufsystem dieses entweder in automatisierter Weise in einem gewünschten Verhältnis aus Kontrastmittel und weiterer apolarer (oder polarer) Komponente(n), gegebenenfalls zeitabhängig, zugemischt und für die Einführung bereitgestellt wird und/oder das Kontrastmittel in der Temperatur eingestellt wird.

Weitere bevorzugte Ausführungsformen der vorliegenden Erfindung sind in den abhängigen Ansprüchen beschrieben.

### KURZE ERLÄUTERUNG DER FIGUREN

Die Erfindung soll nachfolgend anhand von Ausführungsbeispielen im Zusammenhang mit den Zeichnungen näher erläutert werden. Es zeigen:
- Fig. 1: einen postmortalen Mikro-CT Scan einer Maus mit Darstellung der grossen Gefässe;
- Fig. 2: eine bessere Übersicht über die grossen Gefässe des ganzen Körpers nach Entfernung der Rippen und Vorderbeine an der Workstation (virtuelles Skalpell);
- Fig. 3: eine Sichtbarmachung der kleineren Gefäss-Äste durch Veränderung der Fensterung an der Workstation;
- Fig. 4: eine Darstellung des Herzens und der Lebergefässe nach virtuellem Bearbeiten der Daten eines Teilkörperscans einer Maus;
- Fig. 5: eine Darstellung der Hauptäste der Nierengefässe nach virtuellem Ausschneiden des Organes aus einem Teilkörperscan;
- Fig. 6: einen hochauflösenden Scan einer Lungenprobe einer Maus mit 3D-Darstellung kleinster Blutgefässe;
- Fig. 7: eine bessere Sicht auf die Hauptäste der Lungengefässe einer Aufnahme nach Fig. 6 mit dem selben Datensatz durch Veränderung der Fensterung;
- Fig. 8: verschiedene Darstellungsmöglichkeiten einer Niere aus dem Datensatz eines hochauflösenden Einzelorgan-Scans;
- Fig. 9: zeigt die Angio-Injektionsmethode an einer menschlichen Leiche, wobei in a) der Zugang des Kontrastmittels über re A. carotis communis dargestellt ist, und b)-d) unterschiedliche Ansichten der CT-Aufnahmen am Kopf des Untersuchungsobjekts darstellen;
- Fig. 10: zeigt die Angio-Injektionsmethode am Einzelorgan (menschliche Niere), wobei in a) der Zugang des Kontrastmittels über A. renalis dargestellt ist, und b)-d) unterschiedliche Ansichten der CT-Aufnahmen des Scans der Niere darstellen;
- Fig. 11: zeigt die Angio-Injektionsmethode an der unteren Extremität einer menschlichen Leiche, wobei der Zugang über A. femoralis superficialis eröffnet wird und verschiedene Darstellungen des Beines dargestellt sind;
- Fig. 12: zeigt eine schematische Darstellung der Angio-Perfusionsmethode; und
- Fig. 13: zeigt CT-Bilder einer Leiche, die an PAOD (Peripheral Arterial Occlusive Disease) gelitten hatte, wobei in a) und b) die generell sehr schlechte Durchblutung im Bereich des Unterschenkels erkennbar ist, in c) der untere Bereich des Unterschenkels mit PAOD, und in d) - f) die jeweils spezifische Lokalisierung einer Okklusion.

### WEGE ZUR AUSFÜHRUNG DER ERFINDUNG

Das vorgeschlagene Kontrastmittel kann beispielsweise zur mikroskopischen Gefässforschung eingesetzt werden (sog. Microangio), aber ebenfalls zur Angio-Injektionsmethode oder zur Angio-Perfusionsmethode.

Die bevorzugten Mischungen für diese drei Verfahren sind wie folgt:
Microangio: Lipiodol + Decan (Hexadecan oder Tetradecan) zum Beispiel im Verhältnis 1: 4;
Angio-Injektionsmethode: Lipiodol + paraffinum perliquidum + ggf. Decan zum Beispiel im Verhältnis 1: 5 und Decan bis die gewünschte Viskosität erreicht ist;
Angio-Perfusionsmethode: Perfusion mit paraffinum perliquidum eventuell ergänzt mit Decan je nachgewünschter Viskosität, in den bestehenden und weiterlaufenden Kreislauf Bolusinjektion von Lipiodol.

Nachdem wir im Rahmen der Entwicklung einer minimal invasiven postmortalen Angiographie erfolgreich ölige Perfusate und Kontrastmittel an Tierkadavern und menschlichen Leichen eingesetzt haben, wurde auf dieser Basis eine unerwartet vorteilhafte neue Mischung für den Einsatz einer postmortalen Angiographie mittels Micro-CT erstellt. Im Gegensatz zu unseren bisher verwendeten öligen Flüssigkeit kommt es bei dieser neuen Mischung nicht zu Mikroembolien des Kapillarsystems (S. Grabherr at al, A.IR 2006 in press), sondern zur Penetration und damit zur Darstellung dieses Gefässbereiches.

### Bestandteile:

1. Lipiodol ultrafluide® (Guerbert, France)
2. Tetradecan (Tetradecane olefine free, Fluka, Schweiz) oder Hexadecan (Hexadecane, Fluka, Schweiz)

Die Zusammensetzung dieser Komponenten kann variiert werden. Das ölige Kontrastmittel Lipiodol ultrafluide® sorgt für einen hohen Kontrast (ca. 2000 HU), während Decan als Verdünnungsmittel dient, welches die Kapillargängigkeit ermöglicht. Je mehr Decan verwendet wird, umso niedriger wird die Viskosität, und desto kleinere Gefässe können dargestellt werden.

Als bereits erfolgreich eingesetzte Mischungen ist ein Verhältnis von Lipiodol:Decan von 1:4 und 1:6 empfehlenswert.

Vorteile des neuen Kontrastmittels (i.w. für alle drei obengenannten Verfahren):
Praktische Handhabungen: einer der wohl wesentlichsten Vorteile dieser Kontrastmittelmischung ist die praktische Handhabung. Es genügt eine einfache Injektion in den darzustellenden Gefässbereich.

Haltbarkeit der Proben: Da das ölige Kontrastmittel intravasal bleibt, können die Proben nach der Injektion mehrere Tage aufbewahrt und erst dann untersucht werden.

Transportfähigkeit der Proben: Die lange Haltbarkeit der Proben erlaubt den Transport zwischen Labors (z.B. Injektion in Bern, Untersuchung und Analyse in den USA).

Wiederholte Untersuchungsmöglichkeiten: Mehrfache Scans und Analysen einer Probe sind möglich, was bei unklaren Befunden essentiell ist.

3D-Rekonstruktion mit "Zoom in" und virtueller Probenbearbeitung: Mit Hilfe der Untersuchung mittels Micro-CT ist neben der zweidimensionalen auch eine dreidimensionale Rekonstruktion der Daten möglich. Durch Zoom in und virtuelles Schneiden der Proben können beliebige Areale vergrössert, ausgeschnitten und dargestellt werden, ohne die Proben zu zerstören.

Quanitfizierung: Spezielle Software, welche z.B. zur Knochendichtemessung bereits für Micro-CT-Anwendungen existiert, kann sehr leicht zur Quantifizierung der kontrastreichen Gefässe angepasst werden.

Selektive, kaliberabhängige Darstellbarkeit: Je nach Mischungsverhältnis können verschiedene Gefässabschnitte entsprechend ihrem Kaliber dargestellt werden. So können selektiv nur grosse Versorgungsgefässe oder auch Kapillargefässe sichtbar gemacht werden.

Möglichkeit der dynamischen Angiographie: Bei entsprechender Injektionstechnik wird ein präzises Diskriminieren von arterieller, venöser und kapillärer Phase, in Analogie zur klinischen Angiographie erlaubt.

Wiederholbarkeit der Injektion: Bei Durchführung einer dynamischen Angiographie ist aufgrund des fehlenden Verlustes des Kontrastmittels in das umliegende Gewebe eine Ausspülung des Kontrastmittels aus dem Gefässsystem möglich, was eine erneute Injektion ohne Verfälschung der Ergebnisse durch Reste der vorherigen Injektion ermöglicht. Dies ist wichtig, wenn unklare Befunde in einer Phase der dynamischen Angiographie auftreten.

Möglichkeit weiterer Untersuchungen: Die untersuchten Organe können eingebettet und zusätzlich morphologisch untersucht werden (Paraffineinbettung und elektronenmikroskopische Schnittuntersuchung). Dies ist wichtig, wenn die 3D-Struktur mit der Gewebe-Morphologie korreliert und verglichen werden soll.

### Experimenteller Teil:

Mikroangiographie (Microangio):
   bei diesem Verfahren wurde bei einer toten Maus jeweils eine Mischung aus Lipiodol und Decan als Kontrastmittel (KM) in das Gefässsystem injiziert und anschliessend wurde ein Mikro-CT mit einem Gerät des Typs "Siemens Micro-CT-Scanner" durchgeführt.
Vorversuch zur Testung des KM im "Lebend-Scanner:
   KM (Lipiodol : Decan = 1:4) in 0,8 mm Venflon (Verweilkanüle) scannen: KM ist deutlich sichtbar.

### 1. Maus:

Von unten KM (Lipiodol : Decan = 1:4) in die V. cava inf.; Scan nur Oberkörper.

### 2. Maus:

Von unten KM (Lipiodol : Decan = 1:6) in die V. cava inf.; Kein Scan der kompletten Maus; Organentnahme für isolierte Organsscans:
- Herz
- Leber
- Niere
- Kopf

### 3. Maus:

Von unten KM (Lipiodol : Decan = 1:6) in die V. cava inf.; Injektion KM (Lipiodol : Decan = 1:6) in rechte V. saphena; 3 Scans für Ganzkörperdarstellung.

### 4. Maus:

Injektion KM (Lipiodol : Decan = 1:6) in linken Ventrikel; Scan nur Oberkörper.

### 5. Maus:

Injektion KM (Lipiodol : Decan = 1:6) Aorta (wenig) und V. cava inf.; 2 Scans (Kopf und Thorax).

### 6. Maus:

Injektion KM (Lipiodol : Decan = 1:6) von unten in die V. cava inf.; 2 Scans (Kopf und Thorax).

### 7. Maus:

Injektion KM (Lipiodol: Decan = 1:5) in V. porta; Organentnahme für Einzelscans:
- Leberlappen (oberer inkl. Gallenblase)
- Herz
- Re u. li Niere

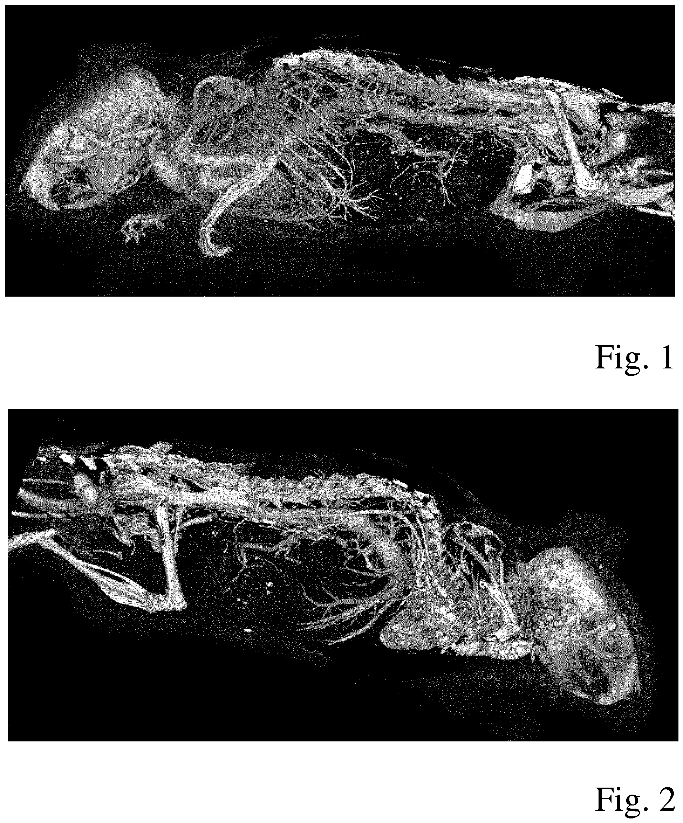

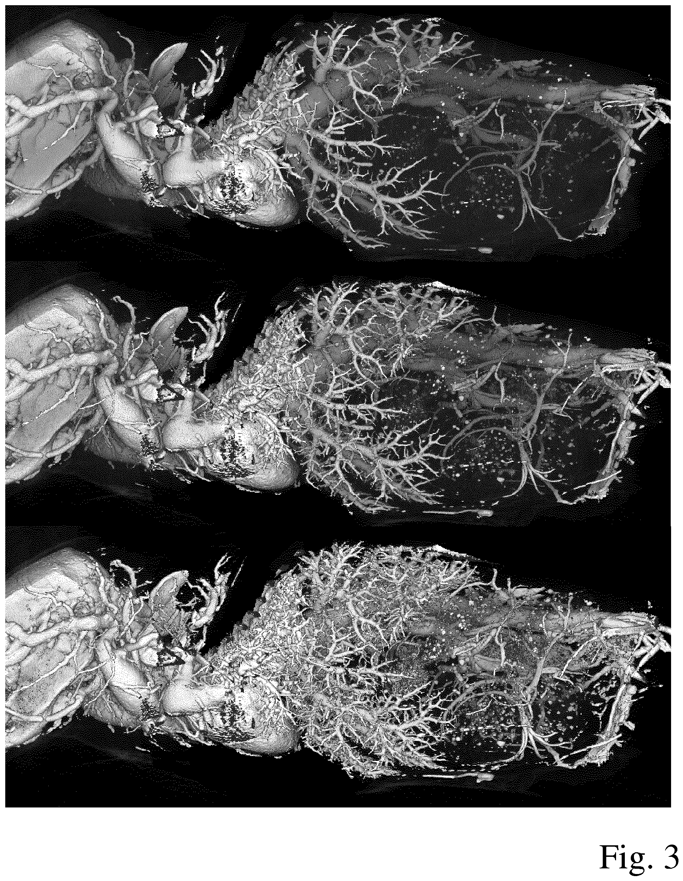

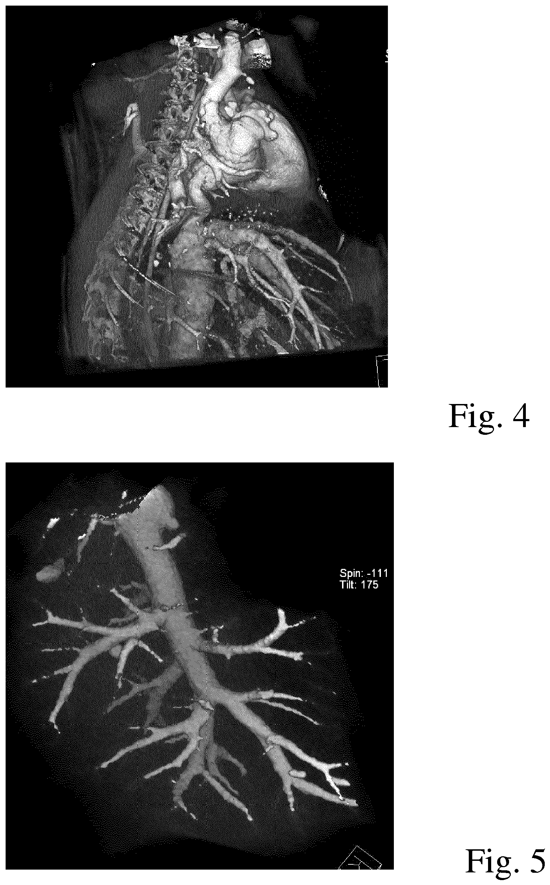

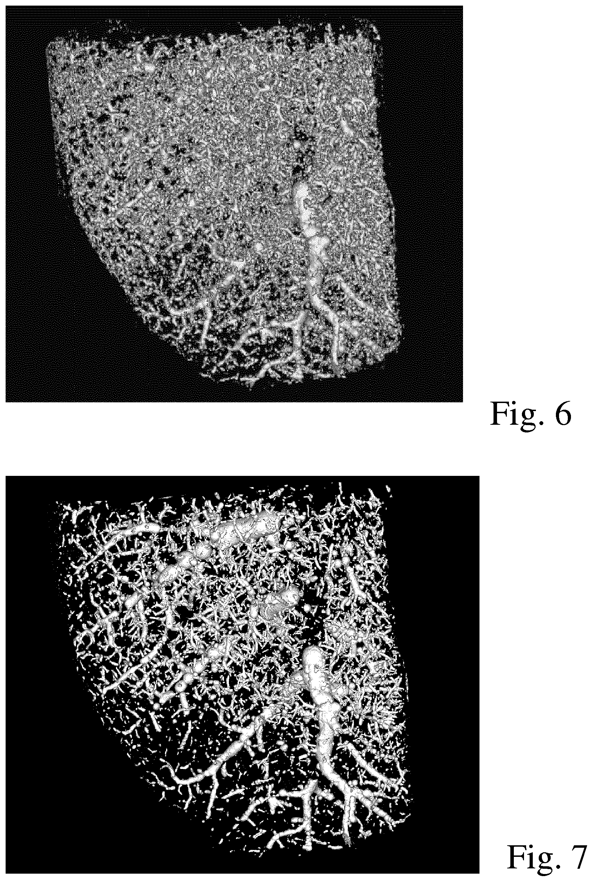

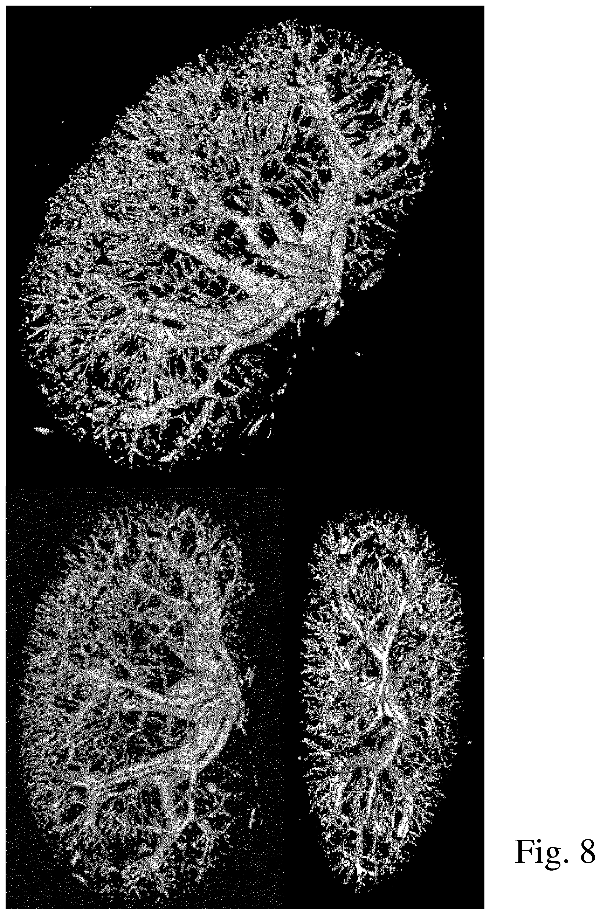

In den Figuren 1-8 sind unterschiedliche Rekonstruktionen aus dem Datensatz der oben angegebenen Micro-CT Scans dargestellt. Aus den Figuren ist ersichtlich, dass das Kontrastmittel einerseits eine hohe Auflösung erlaubt, und andererseits eine hervorragende Penetration selbst in kleinste Gefässsysteme erlaubt.

### Angio-Injektionsmethode:

Hierbei erfolgte eine manuelle Injektion von iodhaltigem Kontrastmittel Lipiodol in seit 2 Jahren formalinfixierte Leichenpräparate, deren Gefässe mit fixiertem Blut verstopft waren.

Vorbereitung mit KM-Verdünnungsserie, wobei Lipiodol : Paraffinöl (jeweils als paraffinum perliquidum) 1:1, 1:5, 1:10 und 1:20 untersucht wurden. Die anfolgenden Aufnahmen wurden mit 1:5 durchgeführt, da dort der beste Kompromiss aus Viskosität, Kontrastwirkung und Penetration in Gefässe gefunden wurde für die angestrebte Visualisierung.

Mischung des Kontrastmittels: Lipiodol : Paraffinöl = 1:5; pro 500 ml dieser Mischung zusätzlich ca. 10 ml Decan. Nach Injektion der Kontrastmittelmischung Multislice-CT mit Rekonstruktion der Daten als MIP und VRT.

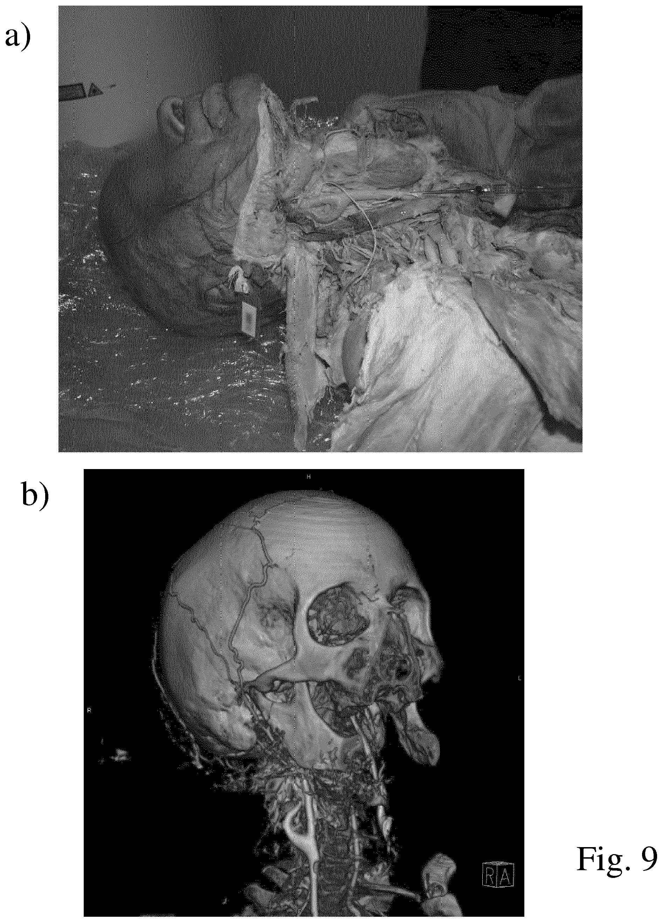

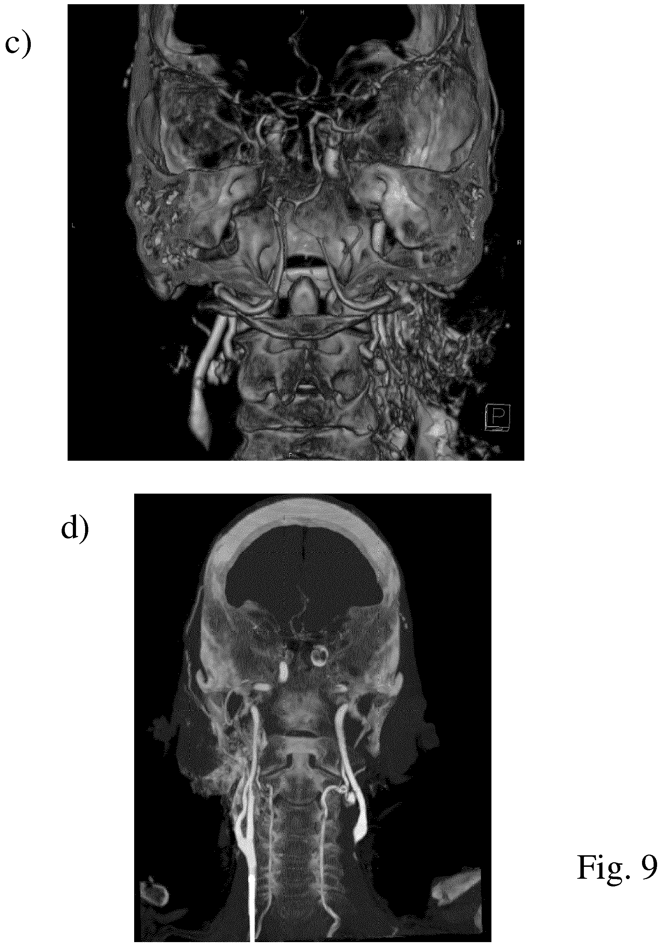

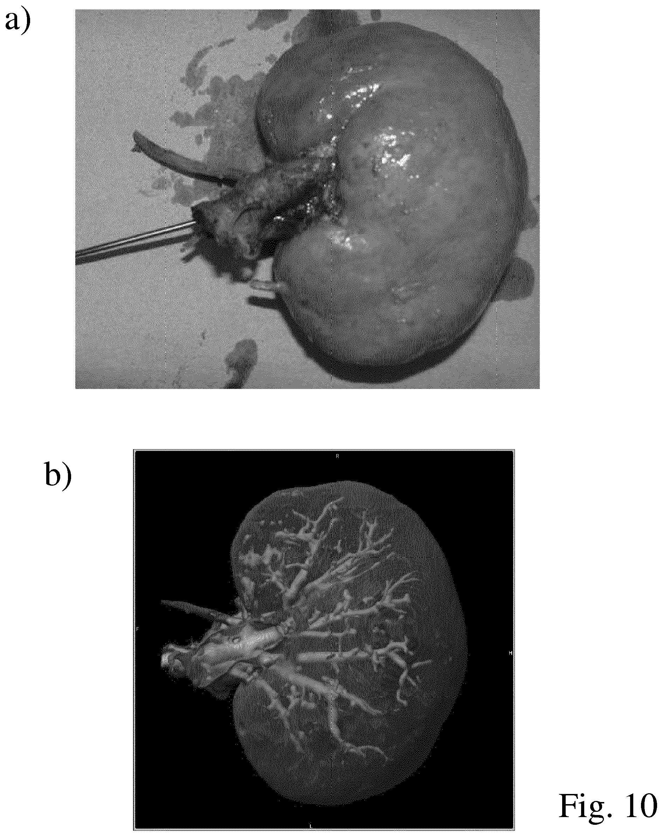

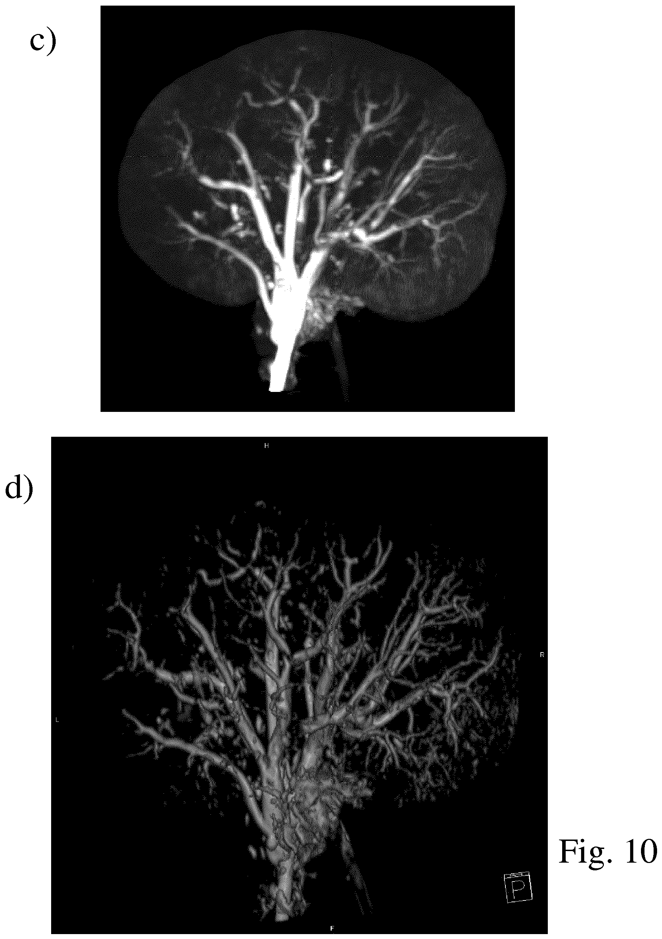

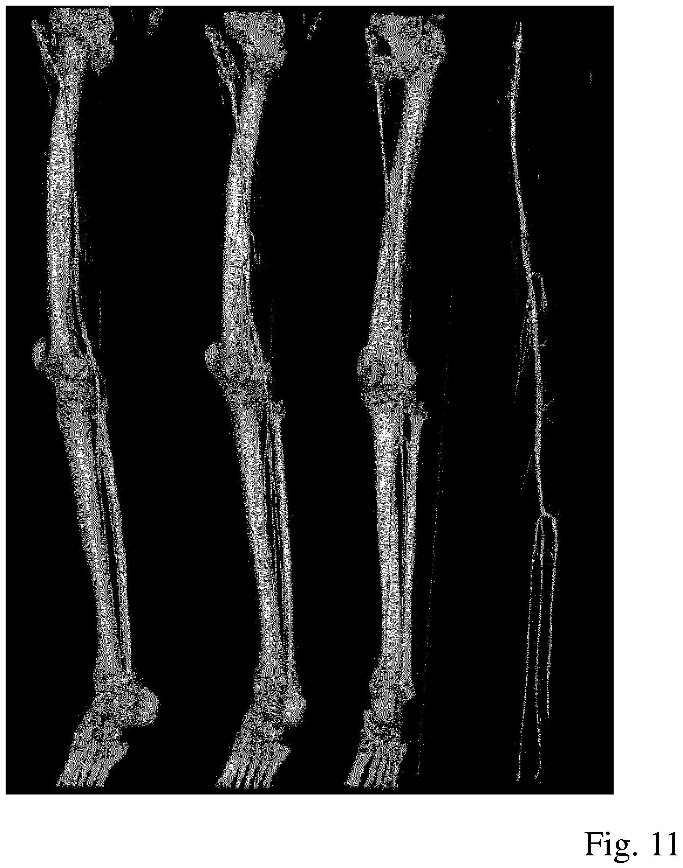

Die Figuren 9-11 zeigen so erhaltene Bilder aus der Angio-Injektionsmethode einerseits vom Kopfbereich (Figur 9), von einer Niere (Figur 10) und von einem Bein (Figur 11), wobei erkennbar ist, wie das verwendete Kontrastmittel einen hervorragenden Kontrast und trotz der zuvor einliegenden Blutgerinnsel eine hervorragende Penetration in die Blutgefässe ermöglicht, ohne dass eine merkliche Extravasation erkennbar ist.

### Angio-Perfusionsmethode:

Angio-Versuch an der menschl. Leiche, Leichnam: Anatomie-Leiche nach Till-Fixierung, bereits Verletzungen vorhanden (Zustand nach Knie-OP links, offene Tibia-Fraktur rechts)

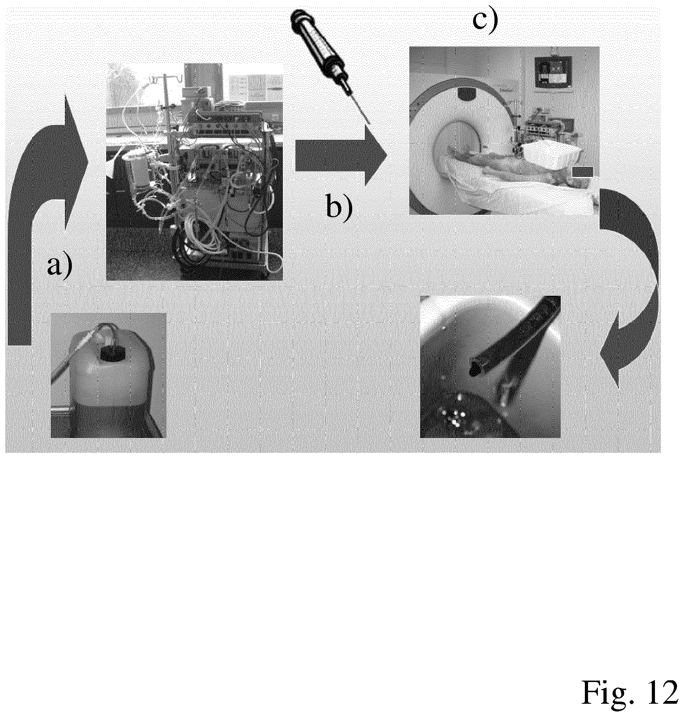

Methode (zu einer schematischen Darstellung vergleiche Figur 12):
- Kanülierung in der rechten Leiste (A. femoralis, V. fomaralis)
- Perfusion mit Hilfe der Herz-Lungen-Maschine (Perfusat: Paraffinöl + Dekan, im Verhältnis ca. 500 ml : 10ml). Perfusionsgeschwindigkeit 5 - 10 ml pro kg Körpergewicht / min. Vgl. a) in Fig. 12.
- Zugabe des Kontrastmittels Lipiodol (40 ml) in das Perfusat (Injektion den Schlauch, der in die Arterie führt, innerhalb einer Zeitspanne von wenigen Sekunden (Bolusinjektion). Vgl. b) in Fig. 12.
- CT-Scans. Vgl. c) in Fig. 12.

Ergebnis: Gelungene Perfusion (Füllen der Varizen an den Beinen, Füllung der A. Carotis); Kontrast im CT.

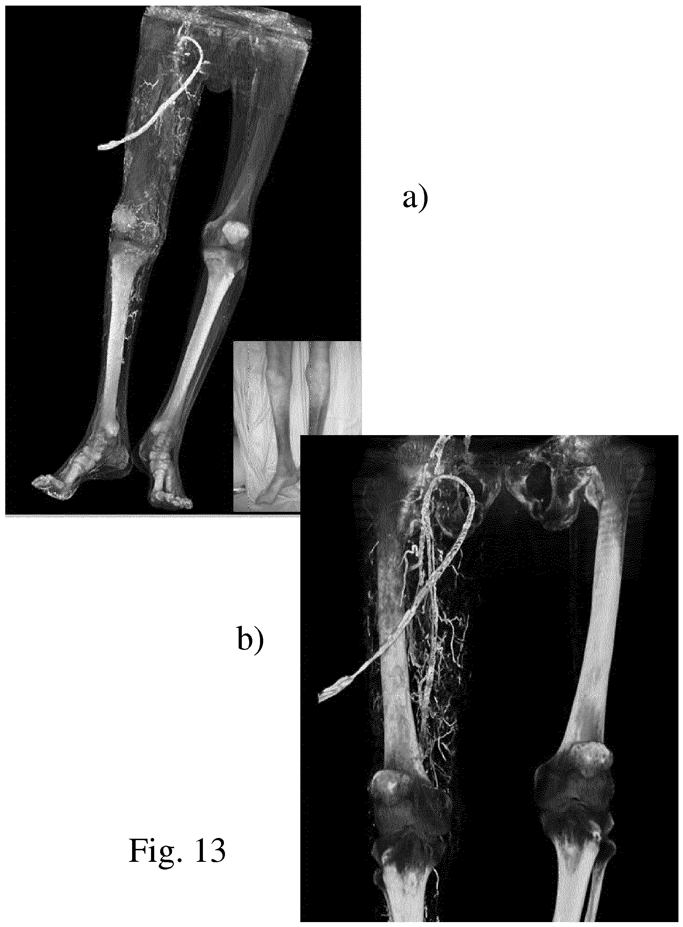

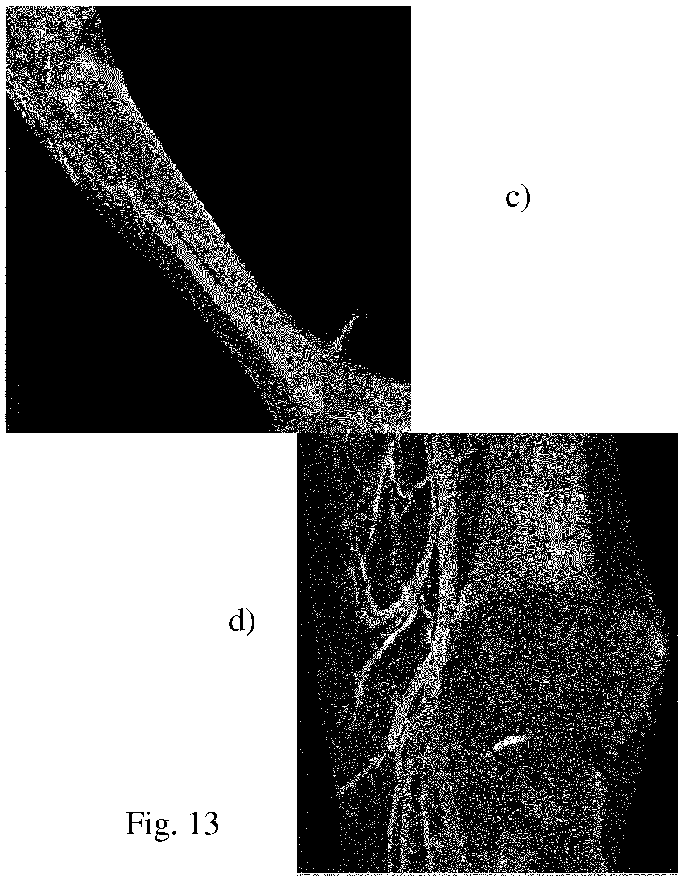

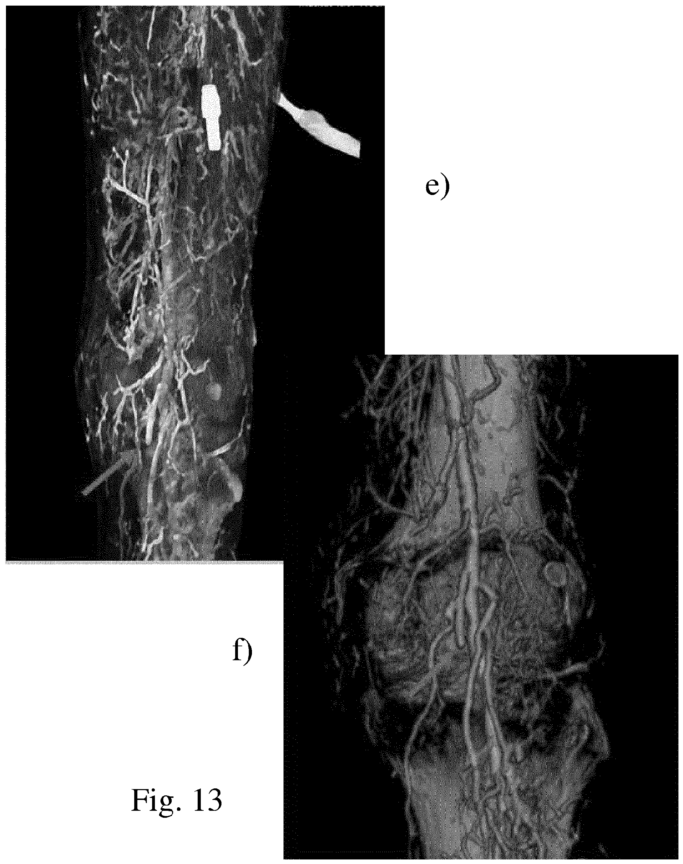

Die Resultate der Perfusion sind in den Figuren 13 a-f erkennbar. Die untersuchte Leiche litt vor dem Tod an PAOD (Peripheral Arterial Occlusive Disease), die Figuren 13 a) und b) zeigen die generell sehr schlechte Durchblutung im Bereich des Unterschenkels, die Figur 13 c) den unteren Berich des Unterschenkels mit PAOD, und die Figuren d) - f) die jeweils spezifische Lokalisierung einer Okklusion.

Kontrastmittel kann entweder als Bolus injiziert oder in grösserer Menge gegeben werden, wobei für dynamische Angiographie ein Bolus innerhalb einer möglichst kurzen Zeitspanne eingebracht werden sollte. Längere Schläuche der Herz-Lungen-Maschine sind von Vorteil für Ganzkörperscan, um die Bewegung des CT-Tisches ausgleichen zu können (typ. ca. 3m).

### Zusammenfassung:

Das neue Kontrastmittel erlaubt eine zuverlässige, rasche, wiederholte Untersuchung und Darstellung von 2D und 3D Gefässarchitektur in Labortieren (z.B. Microangiographie mit MicroCT) sowie postmortal am menschlichen und tierischen Körper (Angio-Injektionsmethode). Zudem erlaubt sie dynamische Bildgebund und Quantifizierung des "Blutverlustes" mit der Angio-Perfusionsmethode. Praktische Handhabung, Quantifikationsmöglichkeiten, sowie exakte Darstellung verschiedenster Gefässabschnitte werden diese Methode als Standarduntersuchung bei der Evaluation genetisch-manipulierter Labortiere, pharmakologischen und toxikologischen Studien, sowie gentechnischen Produkten etablieren. Diese neue Visualisierungs- und Quantifikationsmethode soll die alten, zwar verlässigen, aber sehr zeitaufwendigen und hochspezifischen Techniken ablösen und den Weg für eine ausgedehnte, einfache, praktische und unkomplizierte Anwendung freimachen.

Nachfolgend soll das Maschinenkonzept der postmortalen Angiografie mit Hilfe einer modifizierten Herz-Lungen-Maschine beschrieben werden. Das Maschinenkonzept basiert auf der Grundlage von zwei Angiografiemethoden, die wahlweise stationär (auf einer gerichtsmedizinischen Abteilung) oder mobil durchgeführt werden können. Der Aufbau der Herz-Lungen-Maschine ist deshalb so konzipiert, dass sowohl stationäre, wie auch mobile postmortale Angiografien durchgeführt werden können. Als Basisgerät einer solchen Herz-Lungen-Maschine kann beispielsweise das von der Maquet respektive Jostra vertriebene Gerät mit dem Namen Jostra HL 20 MECC CONSOLE verwendet werden.

Komponenten des Maschinenkonzeptes: Das Maschinenkonzept der modifizierten Herz-Lungen-Maschine beinhaltet sechs Grundkomponenten (Maschinengerüst, Energieversorgung, Antriebseinheit, Steuerungseinheit, Computereinheit sowie Verbrauchsmaterial), die in der Folge einzeln beschrieben werden.

Maschinengerüst: Das Maschinengerüst besteht aus zwei Grundplatten (untere, obere), vier Stahlröhren sowie zwei hinteren Querverstrebungen zur Stabilisierung. Auf der unteren Stahl-Grundplatte ist ein Fahrgestell integriert. Das Fahrgestell mit vier Rädern ist arretierbar und längsachsendrehbar.

Energieversorgung: Prinzipiell wird die modifizierte Herz-Lungen-Maschine über eine externe Quelle mit Energie versorgt. In der Regel wird die Maschine mit 220V oder 110V versorgt. Der Energieumwandler beinhaltet Batterien, welche einen netzstromunabhängigen Betrieb von 120 Minuten gewährleisten (maximale Auslastung).

Antriebseinheit: Die Antriebseinheit besteht aus einer doppelkeilriemen-angetriebenen Rollerpumpe oder Schlauchquetschpumpe. Die Rollerpumpe wird über eine Steckverbindung mit dem Energieumwandler verbunden. Für das mobile Modell ist die genannte zusätzliche Batterieeinheit vorhanden. Auf diese Weise kann nur die Pumpe alleine betrieben werden, was einen zusätzlichen Anwendungsbereich (Single Shot Angiographie) ohne Computereinheit erlaubt, und auch einen statischen Angiografie-Prozess ermöglicht. Für die dynamische Angiographie ist die Rollerpumpe mit der Steuer- und Computereinheit verbunden. Diese Verbindung ermöglicht eine Angiografieform, die sowohl druck- als auch volumenkontrolliert durchgeführt werden kann (siehe unten). Des weiteren ist es möglich, Mittel (z.B. Durchflussheizung etc.) vorzusehen, mit welchen das Zirkulationsmedium vor und/oder nach der Zirkulation erwärmt oder abgekühlt werden kann. Die Rollerpumpe enthält die Möglichkeit der okklusiven Einstellung, um eine druckkontrollierte postmortale Perfusion zu gewährleisten.

Des weiteren kann optional eine Einheit vorgesehen sein, welche das in die Leiche geführte Kontrastmittel aus Ausgangsmaterialien, wie sie oben beschrieben wurden (Kontrastkomponente, weitere apolare Komponente(n) etc.) in gesteuerter Weise automatisch zusammenmischt. Dieses Zusammenmischen kann selbstverständlich auch Zeit-abhängig und Prozess-abhängig durchgeführt werden, um eine wirkliche dynamische Prozessführung bezüglich Kontrastmittelzusammensetzung zu ermöglichen. Die Steuerung dieser Mischeinheit kann durch die unten diskutierte Steuereinheit erfolgen.

Steuereinheit/Bedienermodul: Um die definierten Fragestellungen (Menge, Verschluss u.a) zu beantworten, verfügt die Pumpe über wenigstens vier mögliche Einstellungen (3/16-, 1/4-, 3/8- sowie 1/2 Zoll), über welche mit Hilfe der Determinanten, Tourenzahl und Schlauchdurchmesser, die gesuchten Volumina errechnet und grafisch dargestellt werden können (Ganzkörperperfusionen oder selektive Organperfusionen). Die integrierte Drucksteuerung erlaubt zusätzlich gezielte Aussagen über Verschlussraten definierter Gefässbezirke. Des weiteren kann die Rollerpumpe auch im sogenannten It. oder ml/min. Modus betrieben werden, was zusätzlich definierte Aussagen über Volumenverluste zulässt.

Steuerungseinheit: Die Steuereinheit besteht aus einem bildschirmähnlichen Bedienermodul, sowie elektrischen Modulen, welche an der oberen Grundplatte befestigt sind. Das Bedienermodul der Steuereinheit dient der Netz- und Batteriekontrolle und der Aktivierung der Druck- und Volumenmodi.

Die elektrischen Module werden mit der Computereinheit verbunden. Die elektrischen Module beinhalten eine Druckregistrierung und eine Volumenregistrierung. Die Druckregistrierung besteht aus vier unabhängigen Druckmesseinheiten, die auf jede definierte Druckgrenze eingestellt werden können und somit alle Druckperfusionen ermöglichen und kleinste Druckgradienten erkennen lassen (Blutung, Verschluss). Die Volumenregistrierung besteht aus einer Ultraschallmessung, die einerseits die Applikation des Kontrastmittels und andererseits den Auslauf des Kontrastmittels registriert. Zusätzlich kann die Ultraschallmessung mit dem venösen Reservoir verbunden und somit eine dynamische, kontinuierliche Perfusion ermöglicht werden, da über die Ultraschalldetektion die Flüssigkeit eruiert und quantifiziert wird (siehe Perfusionskonzept).

Die Computereinheit umfasst ein Bedienermodul und dient primär der Datenregistrierung (Drucksensoren, Ultraschall und Pumpenfunktionen der Rollerpumpe), welche mit Hilfe einer Speicherkarte erfasst werden und anschliessend in definierten Programmen visualisiert werden können. Dieser Prozess kann sowohl während als auch nach der postmortalen Perfusion durchgeführt werden, oder als Handprotokoll ausgedruckt werden. Auf der Speicherkarte und/oder der Computereinheit können unterschiedliche Prozessführungen automatisiert vordefiniert werden, das heisst auf der Speicherkarte und/oder der Computereinheit kann Software geladen sein, welche die Prozessführungen, gegebenenfalls nach Einstellung einiger Parameter durch den Benutzer, automatisch steuert.

Des weiteren besteht die Möglichkeit mit Hilfe empirisch erfasster Daten direkt die postmortale Perfusion zu steuern und zu verfeinern. Zusätzlich kann die postmortale Perfusion auf empirisch validierten Daten automatisiert und letztlich standardisiert werden (siehe Validierungskonzept).

Verbrauchsmaterial: Das Verbrauchsmaterial besteht u.a. aus zwei verschiedenen Kunststoff-Schlauchtypen (Silikon, Polyethylen) und einem Hartschalenreservoir sowie zwei Kanülentypen (venös, arteriell) und verschiedenen Konnektoren (die Kanülengrössen und die jeweiligen Kanülentypen sind vom jeweiligen Perfusionsverfahren abhängig). Der Schlauchtyp aus Silikon wird als Antriebsschlauch für die Rollerpumpe verwendet und entspricht i.w. der Länge des Rollerpumpenumfangs. Die Antriebs-Schlauchdimensionen sind variabel und zwischen 3/16 - 1 /2 Zoll im Aussendurchmesser (Ganzkörper- oder selektive Organperfusion). Die restlichen Schlauchverbindungen bestehen aus Polyethylen und haben einen 1/4 Zoll Aussendurchmesser. Das Verbrauchsmaterial wird wie bei einer konventionellen Perfusion angeordnet. Über die venöse Kanüle wird über einen Konnektor der Polyethylenschlauch mit dem Reservoir verbunden als sogenannter venöser Einlass verbunden. Parallel wird über einen Y-Konnektor ein weiterer Schlauch mit der venösen Kanüle verbunden. Dieser Schlauch dient dem Verwurf des primären postmortalen Perfusates (koaguliertes Blut). Der Auslass des Reservoirs wird mit dem Silikonschlauch verbunden, der seinerseits in die Rollerpumpe geführt wird und dabei das Reservoir im Betrieb entleert. Vom Pumpenauslass wird über einen Konnektor der Silikonschlauch mit einem zusätzlichen Polyethylenschlauch verbunden und über einen Konnektor mit Luer-lock (Kontrastmitteleinlass) mit der arteriellen Kanüle verbunden. Das Perfusat wird mit einer speziellen Einfülllinie in das Reservoir geleitet.

Perfusionskonzept, statische klinische Angiografie vs. postmortale dynamische Perfusionsangiografie:
Statische klinische-Angiografie: Heute werden klinisch verschiedene Angiografieverfahren angewendet. Die häufigsten sind die Arteriografie, die Phlebografie, die Koronarangiografie sowie die Varikografie. Ziel ist dabei die Erhaltung eines Angiogramms, welches den gefüllten Gefässinnenraum darstellt und mit welchem verschiedene diagnostische Aussagen bezüglich verschiedener Gefässerkrankungen (KHK, Karotisstenosen, PAVK, Gefässmissbildungen, Thrombosen, Varizen, Gefässverletzungen) gemacht werden können.

Bei der klinischen Angiografie wird das Kontrastmittel mit einem Katheter in das Gefässsystem eingespritzt. Das Herzkreislaufsystem wirkt dabei als "Motor'" bei der Kontrastmittelverteilung. Die Bildgebung ist daher nur in der jeweiligen Kreislaufzeit möglich und führt in der Arteriografie oder in der Koronarangiografie zu einer antegraden Darstellung (d.h. ausschliesslich Messung in Richtung des natürlichen Blutflusses). Auch bei der Phlebo- oder Varikografie wird das Kontrastmittel in der entsprechenden Kreislaufzeit in das venöse Gefässsystem eingeführt und die Bildfolge im antegraden (normalen) Blutfluss aufgenommen. Bei den oben erwähnten Angiografieverfahren handelt es sich also um statische Verfahren, über welche keine quantitativen Flussangaben gemacht werden können, lediglich die Verschlussrate einzelner Gefässe können in % angegeben werden.

Dynamische postmortale Angiografie: Bei der wirklich dynamischen postmortalen Angiografie wird der "Motor" also das Herzkreislaufsystem durch eine Rollerpumpe ersetzt. Durch Wegfallen oder Ersetzen der natürlichen Herzaktivität kann das Gefässsystem antegrad oder erstmals auch retrograd (gegen normalen Blutfluss) gefüllt und dargestellt werden. Gefässpathologien können also von "vorne" oder von "hinten" betrachtet und dargestellt werden. Dies hat relevanten Charakter, so kann beispielsweise ein Koronarthrombus, welcher in der antegraden Bildgebung als subtotale Stenose interpretiert wird, retrograd ausgespült und visualisiert werden. Zusätzlich kann über eine Doppelkanülierung (arteriell-venös) eine schwerwiegende Gefässverletzung sogar quantifiziert werden: Dabei wird über die arterielle Kanüle eine definierte Menge eines definierten Kontrastmittels eingeführt und über eine venöse Kanüle wieder aufgefangen, die Differenz des inflows und outflows ergibt die Menge der Blutverlustes, welcher sich aus den Determinanten Pumpendrehzahl, Schlauchdurchmesser und Zeit errechnet.

Wesentlichste Differenzen der beiden Verfahren: Prinzipiell können alle konventionellen Angiografieverfahren auch mit der postmortalen Perfusion durchgeführt werden. Die fundamental unterschiedlichen Möglichkeiten der beiden Verfahren erlauben aber eine neue Dimension der Gefässdarstellung. Erstmals kann eine Verengung einer Arterie beispielsweise auch distal der Stenose betrachtet und dargestellt werden, was bei der statischen Perfusion am lebenden Körper unmöglich ist.

Ein zweiter wesentlicher Punkt ist der "Motor" der postmortalen Perfusion. Dieser kann vernetzt und über einen Computer betrieben werden. Die Zirkulation des Kontrastmittels kann also fast beliebig beeinflusst werden, was am lebenden Körper selbstverständlich nicht möglich ist. Dies erlaubt nach einer definierten Validierungsphase und dem Abgleich empirisch-evaluierter Daten aus der Herzchirurgie, zum Beispiel eine punktuelle Steuerung der Pumpe und ermöglicht daher eine genaue Quantifizierung der Stenose- oder-Blutungsraten. Als empirisch-evaluierte Daten gelten die Druckgradienten über den verwendeten arteriellen und venösen Kanülen des gesunden Gefässes mit einer definierten Menge Volumen, welche über die Rollerpumpe appliziert wurde. Die dabei gewonnenen Daten gelten als "Normwerte" und werden den erhobenen Daten der postmortalen Perfusion gegenüberstellt. Die Druckdifferenzen werden dann für die genaue Steuerung der Rollerpumpe verwendet.

Des weiteren sei hervorgehoben, dass im Gegensatz zu Messungen am lebenden Körper nicht nur die Zirkulation des Kontrastmittels respektive des gesamten Zirkulationmittels beinahe beliebig im Sinne einer gewünschten Messung eingestellt werden kann, sondern auch andere Parameter. Es kann beispielsweise die Zirkulationsgeschwindigkeit respektive der Zirkulationsdruck auch in Abhängigkeit der Messung respektive der Fortschritts der Messung eingestellt werden, so kann beispielsweise bei einer stossweisen Einführung des Kontrastmittels eine Verlangsamung respektive Druckreduktion in jenem Moment, wo das Analysegerät den Eingang des Kontrastmittels in den in der Hauptsache untersuchten Bereich registriert, veranlasst werden. Es ist möglich bei einem konstanten Druck (das heisst nicht in der natürlichen gepulsten Strömungsweise) Messungen vorzunehmen, und so beispielsweise exakte Daten für die Bedingungen beim maximalen Blutdruck und bei minimalen Blutdruck der Zirkulation zu ermitteln. Des weiteren ist es aber auch möglich, beispielsweise das Zirkulationsmittel und das darin befindliche Kontrastmittel bei unterschiedlichen Temperaturen zirkulieren zu lassen. So kann z.B. die Viskosität aber auch das Strömungsverhalten etc. beeinflusst werden.

Daraus ergeben sich unter anderem zusammenfassend folgende Unterschiede:

| *Klinische Angiografie:* | *Postmortale Perfusion:* |
|---|---|
| Antegrade Perfusion | Retrograde Perfusion |
| Inkonstant (Herz, gepulst, flusskontrolliert) | Konstant (Pumpe, druckgesteuert) |
| Statisch (Kreislaufzeit) | Dynamisch |
| Inflow Verfahren | In- und Outflow-Verfahren |
| Qualitatives Verfahren | Quantitatives Verfahren |

## Patentansprüche

1. Kontrastmittel für die Angiographie, insbesondere zur postmortalen, experimentellen und/oder diagnostischen Angiographie und zur Untersuchung von tierischen oder menschlichen Leichen oder Bestandteilen wie Gliedmassen oder Organen davon, enthaltend eine im wesentlichen ölbasierte, apolare Kontrastkomponente für Röntgenuntersuchungen, welche Kontrastkomponente eine Kontrastkomponentenviskosität im Bereich von 30-100 mPas aufweist, **dadurch gekennzeichnet, dass** die Kontrastkomponente in einer Mischung vorliegt mit wenigstens einer weiteren apolaren Komponente, deren Viskosität geringer ist oder höchstens gleich gross wie die Kontrastkomponentenviskosität, wobei es sich bei der weiteren apolaren Komponente um Paraffinöl handelt.

2. Kontrastmittel nach Anspruch 1, **dadurch gekennzeichnet, dass** es sich bei der weiteren apolaren Komponente um paraffinum perliquidum handelt.

3. Kontrastmittel nach einem der vorhergehenden Ansprüche, **dadurch gekennzeichnet, dass** die Kontrastkomponente eine Viskosität im Bereich von 30 - 80 mPas aufweist, wobei es sich vorzugsweise bei der Kontrastkomponente um ein iodbasiertes oder schwefelbasiertes Röntgenkontrastmittel handelt, insbesondere bevorzugt um eine iodierte oder bromierte Komponente oder insbesondere Öl, beispielsweise um ein Propyliodon, einen Fettsäureethylester von iodiertem Mohnöl, oder Iodipin, wobei es sich insbesondere bevorzugt um Lipiodol ultrafluid handelt.

4. Kontrastmittel nach einem der vorhergehenden Ansprüche, **dadurch gekennzeichnet, dass** das Volumen-Verhältnis von Kontrastkomponente zu weiterer apolarer Komponente im Bereich von 1:1 bis 1:10, bevorzugt im Bereich von 1:2 - 1:5, insbesondere bevorzugt im Bereich von 1:4 - 1:6 liegt.

5. Kontrastmittel nach einem der vorhergehenden Ansprüche, **dadurch gekennzeichnet, dass** die Kontrastkomponente in einer Mischung aus einer ersten weiteren apolaren Komponente und einer zweiten weiteren apolaren Komponente vorliegt, wobei es sich bei der ersten weiteren apolaren Komponente um ein Alkan mit 12-18, bevorzugt mit 14-16 Kohlenstoffatomen, oder 12-16 Kohlenstoffatomen, oder um ein Gemisch von derartigen Alkanen handelt, wobei es sich dabei insbesondere bevorzugt um ein n-Alkan oder ein Gemisch von solchen n-Alkanen handelt, und wobei es sich bei der zweiten weiteren apolaren Komponente um Paraffinöl, insbesondere um paraffinum perliquidum handelt.

6. Kontrastmittel nach einem der vorhergehenden Ansprüche, **dadurch gekennzeichnet, dass** die wenigstens eine weitere apolare Komponente eingefärbt ist, insbesondere mit Sudanrot eingefärbt ist.

7. Verfahren zur angiographischen Untersuchung von tierischen oder menschlichen Leichen oder Organen, Bestandteilen wie Gliedmassen davon, **dadurch gekennzeichnet, dass** ein Kontrastmittel nach einem der vorhergehenden Ansprüche, in das Blutgefässsystem eingebracht wird und anschliessend oder synchron Aufnahmen unter Zuhilfenahme von Röntgenstrahlen, insbesondere eine CT-Aufnahme, vorgenommen werden, wobei vorzugsweise das Kontrastmittel in nicht pulsierender Weise, bevorzugtermassen unter im wesentlichen konstantem oder zeitlich mit wesentlich weniger oder wesentlich mehr als der natürlich möglichen Herzfrequenz variierendem Druck in die Blutgefässe eingebracht wird, und/oder wobei vorzugsweise das Kontrastmittel im Blutgefässsystem wenigstens abschnittsweise im Prozess entgegen der natürlichen Flussrichtung des Blutes zirkuliert wird.

8. Verfahren nach Anspruch 7, **dadurch gekennzeichnet, dass** die Differenz bezüglich Volumen, Druck, Temperatur und/oder Komponentenkonzentration aus in das Kreislaufsystem eingeführtem Kontrastmittel und aus aus dem Kreislaufsystem austretendem Kontrastmittel registriert und in der Analyse bevorzugtermassen in quantitativer Weise mit berücksichtigt wird und/oder dass vorzugsweise das Kontrastmittel bei der Einführung in das Kreislaufsystem in wenigstens einem der folgenden Parameter während und/oder vor der Messung, insbesondere bevorzugt in Abhängigkeit der aufgenommenen Bilddaten, variiert wird: Temperatur, Mischungsverhältnis, Druck, und/oder dass vorzugsweise vor der Einführung von Kontrastmittel das Kreislaufsystem insbesondere bevorzugt unter Verwendung einer apolaren Komponente oder einer NaCl- oder Lactatlösung, gespült wird.

9. Vorrichtung zur Durchführung eines Verfahrens nach einem der Ansprüche 7 oder 8, **dadurch gekennzeichnet, dass** sowohl Mittel zur pumpengetriebenen Einbringung von Kontrastmittel in das Kreislaufsystem vorgesehen sind als auch Mittel zur Aufnahme und quantitativen Erfassung des nach Zirkulation aus dem Kreislaufsystem austretenden Kontrastmittels.

10. Vorrichtung zur Durchführung eines Verfahrens nach einem der Ansprüche 7 oder 8, insbesondere eine Vorrichtung nach Anspruch 9, **dadurch gekennzeichnet, dass** eine Einheit angeordnet ist, mit welcher vor der Einbringung des Kontrastmittels in das Kreislaufsystem dieses entweder in automatisierter Weise in einem gewünschten Verhältnis aus Kontrastmittel und weiterer apolarer Komponente(n) oder der Komponenten davon, gegebenenfalls zeitabhängig, gemischt wird und/oder das Kontrastmittel in der Temperatur eingestellt wird.

11. Verfahren zur angiographischen Untersuchung von tierischen oder menschlichen Leichen oder Organen, Bestandteilen wie Gliedmassen davon, **dadurch gekennzeichnet, dass** zunächst eine weitere apolare Komponente nach einem der Ansprüche 1-6, insbesondere bevorzugt eine Mischung aus Paraffinum perliquidium und Tetradecan und/oder Hexadecan, in das Blutgefässsystem im wesentlichen kontinuierlich eingebracht und darin zirkuliert oder hindurchgeleitet wird, und während eines Zeitabschnitts dieser weiteren apolaren Komponente eine Kontrastkomponente nach einem der Ansprüche 1-6, insbesondere bevorzugt Lipiodol, beigemischt wird, wobei anschliessend, synchron oder abschnittsweise eine Aufnahme unter Zuhilfenahme von Röntgenstrahlen, insbesondere eine CT-Aufnahme, vorgenommen wird.

12. Verwendung eines Kontrastmittels nach einem der Ansprüche 1-6, zur angiographischen Untersuchung von tierischen oder menschlichen Leichen oder Organen, Bestandteilen wie Gliedmassen davon.

## Claims

1. Contrast agent for angiography, especially for postmortem, experimental and/or diagnostic angiography and for examination of animal or human corpses or components, such as extremities or organs thereof, comprising an essentially oil-based, non-polar contrast component for X-ray examinations, said contrast component having a contrast component viscosity in the range of 30-100 mPas, **characterized in that** the contrast component is present in a mixture with at least one further non-polar component of a viscosity which is lower or at the most equal to the contrast component viscosity, wherein the further non-polar component is paraffin oil.

2. Contrast agent according to claim 1, **characterized in that** the further non-polar component is paraffinum perliquidum.

3. Contrast agent according to one of the preceding claims, **characterized in that** the contrast component has a vicosity in the range of 30-80 mPas, wherein the contrast component preferably is an iodine-based or sulphur-based radiographic contrast agent, especially preferably a iodized or brominated component or especially oil, for example a propyliodone, a fatty-acid-ethyl ester of iodized poppy-seed oil, or iodipine, wherein Lipiodol ultrafluid is especially preferred.

4. Contrast agent according to one of the preceding claims, **characterized in that** the volume-ratio of the contrast component to the further non-polar component is in the range of 1:1-1:10, preferably in the range of 1:2-1:5, especially preferably in the range of 1:4-1:6.

5. Contrast agent according to one of the preceding claims, **characterized in that** the contrast component is present in a mixture of a first further non-polar component and a second further non-polar component, wherein the first further non-polar component is an alcane with 12-18, preferably with 14-16 carbon atoms or 12-16 carbon atoms, or a mixture of such alkanes, wherein it especially preferably is an n-alkane or a mixture of such n-alkanes, and wherein the second further non-polar component is paraffin oil, especially paraffinum perliquidum.

6. Contrast agent according to one of the preceding claims, **characterized in that** the at least one further non-polar component is colored, especially colored with Sudan Red.

7. Method for the angiographic examination of animal or human corpses or organs, or components, such as extremities thereof, **characterized in that** a contrast agent according to one of the preceding claims is introduced into the vascular system and that subsequently or synchronously, radiographic images, especially a CT-image, are recorded by the aid of X-rays, wherein preferably the contrast agent is introduced into the blood vessels in a non-pulsing manner, preferably under an essentially constant pressure, or under a pressure which is temporally varying at a substantially lower or substantially higher rate than the naturally possible heart rate and/or wherein preferably the contrast agent is circulated in the vascular system at least section-wise during the process against the natural flow direction of the blood.

8. Method according to claim 7, **characterized in that** the difference of contrast agent introduced into the circulatory system and contrast agent exiting the circulatory system, with respect to volume, pressure, temperature and/or component concentration, is registered and taken into account, preferably quantitatively, in the analysis, and/or that preferably the contrast agent is varied during the introduction into the circulatory system in at least one of the following parameters during and/or prior to the measurement, especially preferably depending on the recorded image data: temperature, mixing ratio, pressure, and/or that preferably prior to the introduction of contrast agent, the circulatory system is rinsed, especially preferably by using a non-polar component or a NaCl- or lactate-solution.

9. Device for carrying out a method according to one of claims 7 or 8, **characterized in that** means for the pump-driven introduction of contrast agent into the circulatory system are provided, as well as means for receiving and quantitatively registering the contrast agent exiting the circulatory system after circulation.

10. Device for carrying out a method according to one of claims 7 or 8, especially a device according to claim 9, **characterized in that** a unit is provided, with which, prior to the introduction of the contrast agent into the circulatory system, the contrast agent is mixed, either in an automated manner at a desired ratio of contrast agent and one or more further non-polar or polar components, respectively, or the components thereof, possibly in a temporally dependent manner, and/or that the contrast agent is adjusted in the temperature.

11. Method for the angiographic examination of animal or human corpses or organs, or components, such as extremities thereof, **characterized in that** first a further non-polar component according to one of claims 1-6, especially a mixture of paraffinum perliquidum and tetradecane and/or hexadecane, is introduced into the vascular system in an essentially continuous manner, and is circulated therein or conducted through it, and that, during a time span of this non-polar component a contrast component according to one of claims 1-6, especially preferably Lipiodol, is synchronously or section-wise admixed, wherein subsequently a radiograph image by the aid of X-rays, especially a CT-image, is recorded.

12. Use of a contrast agent according to one of claims 1-6, for the angiographic examination of animal or human corpses or organs, or components, such as extremities thereof.

## Revendications

1. Agent de contraste pour l'angiographie, en particulier pour l'angiographie post-mortem, expérimentale et/ou diagnostique et pour l'examen de cadavres animaux ou humains, ou de constituants, tels que des membres ou des organes de ceux-ci, contenant un composant de contraste apolaire, essentiellement à base d'huile pour des examens aux rayons X, le composant de contraste présentant une viscosité de composant de contraste dans la plage de 30-100 mPas, **caractérisé en ce que** le composant de contraste se trouve dans un mélange avec au moins un autre composant apolaire, dont la viscosité est inférieure ou au maximum égale à la viscosité du composant de contraste, l'autre composant apolaire étant de l'huile de paraffine.

2. Agent de contraste selon la revendication 1, en ce que qu'il s'agit, pour l'autre composant apolaire, de paraffinum perliquidum.

3. Agent de contraste selon l'une quelconque des revendications précédentes, **caractérisé en ce que** le composant de contraste présente une viscosité dans la plage de 30-80 mPas, le composant de contraste étant de préférence un agent de contraste pour rayons X à base d'iode ou à base de soufre, en particulier de préférence un composant ou en particulier une huile iodé(e) ou bromé(e), par exemple d'une propyliodone, d'un ester éthylique d'acide gras d'huile d'oeillette iodée, ou d'Iopidine et en particulier de préférence de Lipiodol ultrafluide.

4. Agent de contraste selon l'une quelconque des revendications précédentes, **caractérisé en ce que** le rapport volumique du composant de contraste à l'autre composant apolaire se situe dans la plage de 1:1 à 1:10, de préférence dans la plage de 1:2-1:5, en particulier de préférence dans la plage de 1:4-1:6.

5. Agent de contraste selon l'une quelconque des revendications précédentes, **caractérisé en ce que** le composant de contraste se trouve dans un mélange avec un premier autre composant apolaire, et un deuxième autre composant apolaire, le premier autre composant apolaire étant un alcane comprenant 12-18, de préférence 14-16 ou 12-16 atomes de carbone, ou un mélange de tels alcanes, **en ce qu'**il s'agit de préférence d'un n-alcane ou un mélange de ceux-ci n-alcanes, et **en ce qu'**il s'agit pour le deuxième autre composant apolaire de huile de paraffine, en particulier de préférence de paraffinum perliquidum.

6. Agent de contraste selon l'une quelconque des revendications précédentes, **caractérisé en ce que** l'au moins un autre composant apolaire est coloré, en particulier avec rouge Soudan.

7. Procédé pour l'examen angiographique de cadavres animaux ou humains, ou d'organes, de constituants tels que des membres de ceux-ci, **caractérisé en ce qu'**un agent de contraste selon l'une quelconque des revendications précédentes est introduit dans le système vasculaire et on réalise ensuite ou de manière synchrone des clichés à l'aide de rayons X, en particulier un cliché de TDM, l'agent de contraste étant de préférence introduit dans les vaisseaux dans un mode non pulsé, plus préférablement sous une pression essentiellement constante ou variant dans le temps de manière sensiblement moins ou sensiblement plus que la fréquence cardiaque naturellement possible et/ou l'agent de contraste étant de préférence mis en circulation dans le système vasculaire dans le procédé au moins par sections en sens inverse du sens d'écoulement normal du sang.

8. Procédé selon la revendication 7, **caractérisé en ce que** la différence en ce qui concerne le volume, la pression, la température et/ou la concentration en composants entre l'agent de contraste introduit dans le système circulatoire et l'agent de contraste sortant du système circulatoire est enregistrée et prise en considération dans l'analyse, de préférence de manière quantitative, et/ou **en ce qu'**au moins un des paramètres de l'agent de contraste est de préférence varié, lors de l'introduction dans le système circulatoire, pendant et/ou avant la mesure, en particulier de préférence en fonction des données d'images enregistrées : température, rapport de mélange, pression, et/ou **en ce que** le système circulatoire est de préférence rincé avant l'introduction de l'agent de contraste, en particulier de préférence en utilisant un composant apolaire ou une solution de NaCl ou de lactate.

9. Dispositif pour la réalisation du procédé selon l'une quelconque des revendications 7 ou 8, **caractérisé en ce que** des moyens pour l'introduction d'agent de contraste dans le système circulatoire, actionné du pompe, sont prévus, tant que des moyens pour le réception et l'enregistrement quantitatif de l'agent de contraste émergent du système circulatoire.

10. Dispositif pour la réalisation du procédé selon l'une quelconque des revendications 7 ou 8, en particulier un dispositif selon revendication 9, **caractérisé en ce qu'**une entité et présent, avec celle l'agent de contrast ou bien es mélangé, avant l'introduction de l'agent de contraste dans le système circulatoire, d'une manière automatisé dans une proportion desirée d'agent de contraste et d'autre(s) composant(s) apolaire(s) ou de ses composants, éventuellement en fonction du temps, ou bien/et la temperature du l'agent de contraste est adaptée.

11. Procédé pour l'examen angiographique de cadavres animaux ou humains, ou d'organes, de constituants tels que des membres de ceux-ci, **caractérisé en ce qu'**on introduit d'abord un autre composant apolaire selon l'une quelconque des revendications 1-6, en particulier un mélange de paraffinum perliquidum et tétradécane et/ou hexadécane, dans le système vasculaire de manière essentiellement continue et on le met en circulation ou on le guide à travers celui-ci, et pendant un intervalle de temps on mélange à ce composant apolaire un composant de contraste selon l'une quelconque des revendications 1-6, en particulier de préférence du Lipiodol ou un composant de contraste polaire, un cliché étant réalisé consécutivement, de manière synchrone ou par sections à l'aide de rayons X, en particulier un cliché de TDM.

12. Utilisation d'un agent de contraste selon l'une quelconque des revendications 1-6 pour l'examen angiographique de cadavres animaux ou humains, ou d'organes, de constituants tels que des membres de ceux-ci.
